(19) Europäisches Patentamt
European Patent Office
Office européen des brevets

(11) Veröffentlichungsnummer: **0 039 483**
**B1**

(12) # EUROPÄISCHE PATENTSCHRIFT

(45) Veröffentlichungstag der Patentschrift: **21.11.84**

(51) Int. Cl.³: **C 07 D 277/68**

(21) Anmeldenummer: **81103258.0**

(22) Anmeldetag: **30.04.81**

(54) **Verfahren zur Herstellung von 2-Hydroxybenzthiazolen.**

(30) Priorität: **05.05.80 DE 3017153**

(43) Veröffentlichungstag der Anmeldung: **11.11.81 Patentblatt 81/45**

(45) Bekanntmachung des Hinweises auf die Patenterteilung: **21.11.84 Patentblatt 84/47**

(84) Benannte Vertragsstaaten: **BE CH DE FR GB IT LI**

(56) Entgegenhaltungen: **DE - A - 2 812 940**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr., Heinrich-Bleicher-Strasse 40, D-6000 Frankfurt am Main 50 (DE)**

EP 0 039 483 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung liegt auf dem technischen Gebiet der Zwischenprodukte und betrifft insbesondere ein verbessertes Verfahren zur Herstellung von 2-Hydroxybenzthiazolen.

2-Hydroxybenzthiazole (bzw. 2(3H)-Benzthiazolone) sind technisch wichtige Zwischenprodukte, die beispielsweise aus der deutschen Offenlegungsschrift 2 924 712, aus der britischen Patentschrift 1 317 561 und aus der US-Patentschrift 3 775 333 bekannt sind. Die Synthese dieser Verbindungsklasse ist technisch bisher nicht befriedigend gelöst. Entweder ist sie mit schwerwiegenden ökologischen Problemen verbunden, beispielsweise, wenn von Thiophenolen oder deren Derivaten ausgegangen wird, oder die in der Synthese eingesetzten Ausgangsverbindungen sind technisch schlecht zugänglich, wie beispielsweise die 2-Halogen-, 2-Alkoxy- oder 2-Alkylsulfonyl-benzthiazole.

Im einzelnen sind folgende Herstellungsmöglichkeiten bekannt geworden:

a) Cyclisierung von o-Aminothiophenolen mit Phosgen, Kohlenoxidsulfid, Harnstoff oder Cyanat (J. Chem. Soc. 1930, 128—135; US-PS 2 915 525; DE-OS 2 131 366; J. Chem. Soc. 1962, 230).
b) Katalytisch-reduktive Cyclisierung von o-Nitrothiophenolen mit Kohlenmonoxid (J. Chem. Soc. 1927, 2738).
c) Umsetzung von o-Nitrochlorbenzolen mit Thioglykolsäure und Cyclisierung des gebildeten Thioäthers mittels Essigsäureanhydrid (Egypt. J. Chem. 16, 335 (1973)).
d) Oxidation von 2-Mercaptobenzthiazolen zu Benzthiazol-2-sulfonaten und deren saure Hydrolyse (Deutsche Patentschrift 615 131; US-PS 2 179 987; J. Org. Chem. 27, 477 (1962)).
e) Spaltung von Benzthiazolyl-alkyl-thioäthern mit Alkali oder alkoholischer Jodlösung (J. Chem. Soc. 1929, 470, und 1942, 304).
f) Umsetzung von 2-Halogenbenzthiazolen mit Wasser, Formamid oder Phenol und gegebenenfalls nachfolgender Hydrolyse oder Hydrogenolyse (Chem. Ber. 12, 1128 (1879), und 13, 10 (1880); J. Org. Chem. 30, 3618 (1965); US-PS 3 658 835).
g) Ätherspaltung von 2-Alkoxy-benzthiazolen (J. Am. Chem. Soc. 71, 3349 (1949), und 74, 1081 (1952)).
h) Saure Hydrolyse von 2-Alkylsulfonyl-benzthiazolen (J. Chem. Soc. 1949, 3311).

Die Verfahrensvarianten a) bis e) gehen bezüglich der Ausgangsverbindungen oder deren Vorstufen von Mercaptoverbindungen aus oder sind davon abhängig. Die Synthese, Handhabung und Entsorgung solcher Mercaptoverbindungen sind mit erheblichen ökologischen Problemen behaftet; für diese Mercaptoverbindungen fehlen in der Regel, von den substituentenfreien Grundkörpern abgesehen, gut zugängliche Ausgangsprodukte, weswegen der Variationsrahmen in diesen bekannten Verfahrensweisen eng begrenzt ist.

Die obengenannten Verfahrensvarianten f) bis h) sind dagegen praktisch nur von wissenschaftlichem Interesse, da für deren Ausgangsverbindungen keine technisch realisierbaren Synthesemöglichkeiten bekannt sind, die annehmbare Ausbeuten liefern. Vielmehr werden die für diese Verfahrensweisen beschriebenen Ausgangsprodukte in üblicher, meist optimaler Weise aus den hier zu synthetisierenden 2-Hydroxybenzthiazolen aufgebaut.

Es bestand somit ein erhebliches Interesse an einem allgemein anwendbaren, technisch realisierbaren und verbesserten Verfahren zur Herstellung von 2-Hydroxybenthiazolen.

Mit der vorliegenden Erfindung wurde nunmehr ein Verfahren zur Herstellung von 2-Hydroxybenzthiazolen gefunden, das dadurch gekennzeichnet ist, daß man eine 2-Amino-benzthiazol-Verbindung mit einem Alkali- oder Erdalkalihydroxid in zumindest stöchiometrischer Menge in einem alkalibeständigen Löse- oder Verdünnungsmittel in Abwesenheit von Wasser oder lediglich in Anwesenheit von höchstens 2,5 Gew.-%, vorzugsweise höchstens 0,5 Gew.-% Wasser in der Reaktionsmischung, bezogen auf die Reaktionsmischung, behandelt, das sich bildende Alkali- oder Erdalkalisalz der ortho-Mercapto-N-phenylharnstoff-Verbindung, gegebenenfalls ohne deren Zwischenisolierung, durch Säure in deren ortho-Thiophenol-harnstoff-Verbindung überführt und diese mittels Säure bei einer Temperatur zwischen 40 und 100° C zur 2-Hydroxy-benzthiazol-Verbindung cyclisiert.

Die Reaktion läuft nach folgendem Schema:

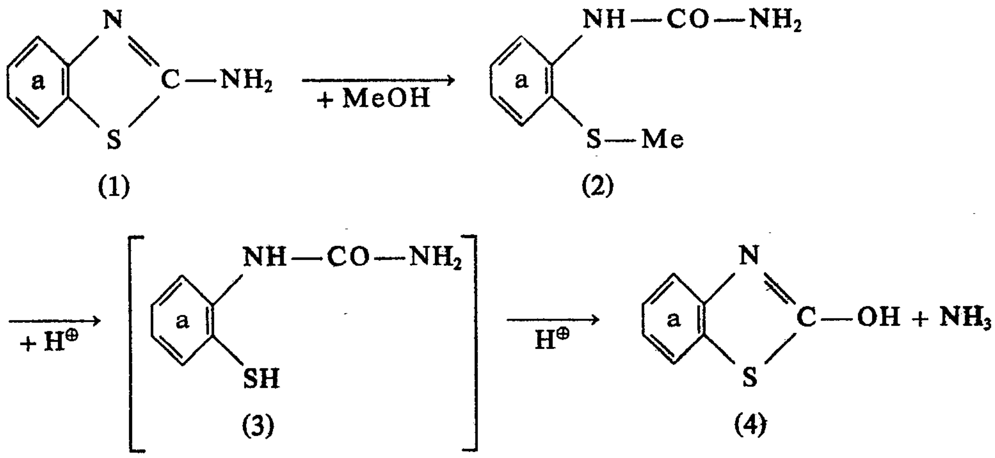


(1) (2) (3) (4)

(In den Formeln (1) bis (4) steht Me für das Äquivalent eines Alkalimetalls oder Erdalkalimetalls, und der Benzolkern a kann weitere Substituenten tragen; der bei der Cyclisierung frei werdende Ammoniak wird durch die Säure als Ammoniumion gebunden.)

Die zur Ringöffnung in die Reaktion eingesetzten Alkali- oder Erdalkalihydroxide MeOH sind bevorzugt Barium-, Calcium- und Kaliumhydroxid, insbesondere jedoch Natriumhydroxid. Die zur Cyclisierung der Zwischenverbindungen der Formel (2) in die Reaktion eingesetzten Säuren sind vorzugsweise Mineralsäuren, wie Salzsäure, Schwefelsäure und Phosphorsäure.

Alkalibeständige Löse- und Verdünnungsmittel, die wasserfrei sein sollten und in denen erfindungsgemäß die Ringspaltung der 2-Aminobenzthiazole, gegebenenfalls auch die Cyclisierung der daraus entstehenden Mercaptophenylharnstoffe, durchgeführt wird, sind insbesondere ein- oder mehrwertige aliphatische Alkohole, wie beispielsweise Alkanole von 1 bis 6 C-Atomen, Alkanglykole von 2 bis 5 C-Atomen, Alkantriole von 3 bis 8 C-Atomen, höhermolekulare Alkanpolyole und niedere Monoalkyläther der genannten Glykole, Triole und Polyole.

Es war äußerst überraschend, daß die technisch gut zugänglichen 2-Aminobenzthiazol-Verbindungen (siehe die DE-OS 2 801 991) auf einfache Weise und in hoher Ausbeute und Reinheit unter Verwendung technisch leicht zugänglicher und billiger Chemikalien, die keine Abwasserprobleme aufwerfen, in 2-Hydroxybenzthiazol-Verbindungen übergeführt werden können. Aus Chem. Ber. 13, 20 (1880) ist nämlich bekannt, daß 2-Aminobenzthiazole bei der Behandlung mit geschmolzenem Alkali unter Zerstörung des heterocyclischen Ringes irreversibel zu o-Amino-thiophenolaten und Ammoniak sowie Alkalicarbonaten gespalten werden. Mit der vorliegenden Erfindung wurde dagegen eine schonende Ringöffnung erreicht. Bei dem erfindungsgemäßen Verfahren entweicht praktisch kein Ammoniak, und man erhält in hoher, oft nahezu quantitativer Ausbeute als Zwischenverbindungen die Alkali- oder Erdalkalisalze der entsprechenden o-Mercaptophenylharnstoffe (s. obige Formel (2)). Diese stabilen Mercaptide der Formel (2) lassen sich dann direkt oder nach deren Zwischenisolierung durch eine Säurebehandlung in die 2-Hydroxybenzthiazole überführen.

Diese säurekatalytische Cyclisierung der Verbindungen der Formel (2) bzw. (3) zu den 2-Hydroxybenzthiazolen der Formel (4) ist zwar aus J. Chem. Soc. 1962, 230, bekannt, jedoch blieb dieser Cyclisierungsreaktion die technische Ausführung bisher verschlossen, weil die Herstellung der Ausgangsverbindungen der Formel (3) nur durch Umsetzung von ortho-Amino-thiophenolen mit überschüssigem Natriumcyanat möglich war, für die die obenerwähnten ökologischen und synthetischen Probleme bestanden.

Mit der Auffindung des neuen Verfahrens zur Herstellung der Schlüsselverbindung der Formel (2) bzw. (3) ist es nunmehr möglich, in einfacher, technisch vorteilhafter Weise aus leicht zugänglichen Vorprodukten 2-Hydroxybenzthiazol-Verbindungen herzustellen.

Von den als Ausgangsverbindungen dienenden 2-Aminobenzthiazol-Verbindungen kommen bevorzugt solche in Frage, die in der deutschen Offenlegungsschrift 2 801 991 beschrieben sind. Besonders bevorzugt werden nach dem erfindungsgemäßen Verfahren die 2-Hydroxybenzthiazole der allgemeinen Formel (4a)

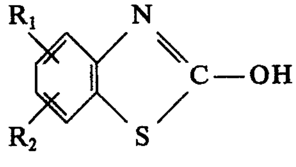

(4a)

in welcher $R_1$ und $R_2$ gleich oder verschieden voneinander sind und jedes ein Wasserstoffatom, eine Alkylgruppe, bevorzugt niedere Alkylgruppe, eine Alkoxygruppe, bevorzugt eine niedere Alkoxygruppe, ein Halogenatom, bevorzugt ein Fluor- oder Chloratom, eine Hydroxy- oder Sulfogruppe bedeuten, hergestellt, wobei erfindungsgemäß von 2-Aminobenzthiazolen der allgemeinen Formel (1a)

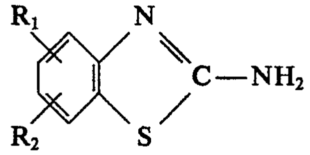

(1a)

in welcher $R_1$ und $R_2$ die obengenannten Bedeutungen haben, ausgegangen wird.

Das erfindungsgemäße Verfahren der Ringspaltung der 2-Aminobenzthiazole zu den o-Mercaptophenylharnstoffen soll in wasserfreiem Medium durchgeführt werden, um hohe Ausbeuten zu gewährleisten. Die Umsetzung kann zwar auch in Gegenwart von geringen Mengen Wasser, die in den Löse- oder Verdünnungsmitteln vorhanden sein können, durchgeführt werden, jedoch verringert sich hierdurch die Ausbeute und gegebenenfalls Reinheit des Verfahrensproduktes. Die Forderung in dem erfindungsgemäßen Verfahren geht deshalb dahin, die Verfahrensweise der Ringspaltung der 2-Aminobenzthiazole unter weitgehendem Ausschluß von Wasser auszuführen.

Die Reaktionstemperatur des erfindungsgemäßen Verfahrensschrittes zur Herstellung der Verbindungen der Formel (2) kann im Bereich zwischen 80 und 200° C gewählt werden; vorzugsweise arbeitet man bei einer Temperatur zwischen 120 und 160° C. Bei Verwendung von niedrigsiedenden Löse- oder Verdünnungsmitteln, wie beispielsweise Alkanolen von 1 bis 3 C-Atomen oder niederen Glykol-monoalkyläthern, arbeitet man deshalb im geschlossenen System unter Druck.

Das bei der Ringspaltungsreaktion verwendete Alkali- oder Erdalkalihydroxid wird in zumindest stöchiometrischer Menge eingesetzt. Zur Vermeidung von Nebenreaktionen hat sich ein Überschuß von einer 1- bis 4-fach, vorzugsweise einer 2- bis 3-fach molaren Menge Alkali- oder Erdalkalihydroxid als günstig erwiesen.

Die erfindungsgemäß hergestellten Alkali- bzw. Erdalkalimetallsalze der ortho-Mercapto-phenylharnstoffe werden anschließend, gegebenenfalls nach Abtrennung aus dem Reaktionsmedium durch Entfernung des Löse- oder Verdünnungsmittels, durch Säure in die ortho-Thiophenol-harnstoff-Verbindung übergeführt. Hierzu sind äquivalente Mengen an Säure erforderlich. Die anschließende Cyclisierung dieser Thiophenolharnstoffe zu den 2-Hydroxybenzthiazolen kann sowohl mit katalytischen als auch mit überschüssigen Mengen an Säure erfolgen. Die Cyclisierungsreaktion kann in einem Löse- oder Verdünnungsmittel durchgeführt werden, vorteilhaft jedoch in dem wäßrigen Medium der angewandten Säure. Die Cyclisierungsreaktion erfolgt bei einer Temperatur zwischen 40 und 100° C, bevorzugt 60 und 90° C; sie ist in kurzer Zeit beendet.

Das erfindungsgemäße Verfahren kann in der Weise durchgeführt werden, daß man eine 2-Aminobenzthiazol-Verbindung, beispielsweise der allgemeinen Formel (1) oder (1a), in einem wasserfreien oder praktisch wasserfreien Lösemittel, wie Äthanol, Isobutanol, 1,2-Dihydroxypropan oder 1,3-Dihydroxypropan, bevorzugt Äthylenglykol, Glycerin, Äthylenglykol-monomethyläther oder Äthylenglykol-monoäthyläther, mit dem festen Alkali- oder Erdalkalihydroxid, bevorzugt Natriumhydroxid, unter Rühren auf eine Temperatur zwischen 120 und 160° C erhitzt und innerhalb dieses Temperaturbereiches die Reaktion mehrere Stunden weiterführt. Die vollständige Umsetzung kann durch chromatographische Prüfung auf Ausgangsverbindung kontrolliert werden. Während dieser Ringöffnungsreaktion entweicht praktisch kein Ammoniak. Das Alkali- oder Erdalkalisalz der gebildeten o-Mercaptophenylharnstoff-Verbindung scheidet sich kristallin aus dem Reaktionsgemisch ab und kann durch Filtration von der gegebenenfalls recyclisierbaren Mutterlauge abgetrennt und der anschließenden Cyclisierungsreaktion zugeführt werden. Das gebildete Salz der o-Mercaptophenylharnstoff-Verbindung kann jedoch auch im Reaktionsmedium belassen werden und direkt durch Zugabe einer mindestens stöchiometrischen Menge, bezogen auf das eingesetzte Alkali- oder Erdalkalihydroxid, an Mineralsäure in die freie o-Mercaptophenylharnstoff-Verbindung überführt werden, die sich, beispielsweise nach Zugabe von Wasser, durch Filtration isolieren läßt. Infolge der Instabilität dieser freien o-Mercaptophenylharnstoff-Verbindungen ist es jedoch vorteilhaft, diese oder deren Salz direkt im sauren Medium durch kurzzeitiges Erhitzen zur gewünschten 2-Hydroxybenzthiazol-Verbindung zu cyclisieren. In diesem letzteren Falle erfolgt die Isolierung des Endproduktes entweder durch Filtration aus dem eingesetzten Lösemittel, gegebenenfalls nach weiterer Verdünnung mit Wasser, oder durch Abblasen des Lösemittels und nachfolgender mechanischer Trennung der erhaltenen Suspension, beispielsweise mittels Filterpresse, Separator oder Zentrifuge. — Geht man zur Cyclisierung von dem isolierten Salz der o-Mercaptophenylharnstoff-Verbindung bzw. der isolierten freien Mercaptoverbindung aus, so wird dieses in Wasser, das ein weiteres Löse- oder Verdünnungsmittel enthalten kann, gelöst oder suspendiert; nach Zugabe von Säure bis zu einem sauren pH-Wert wird dieses Gemisch auf 40 bis 100° C, vorzugsweise 60 bis 90° C, erhitzt.

In einigen Fällen kann auch so verfahren werden, daß man nach der Ringöffnung das Löse- oder Verdünnungsmittel aus der alkalischen Reaktionsmischung abdestilliert oder mit Wasserdampf abbläst und das hierbei erhaltene Salz der o-Mercaptophenyl-harnstoff-Verbindung bzw. deren wäßrige Lösung mit Säure, vorzugsweise wäßriger Säure, bis zum sauren pH-Bereich versetzt und sodann im

oben angegebenen Temperaturbereich erhitzt.

Das erfindungsgemäße Verfahren geht von technisch vorteilhaft zugänglichen Ausgangsverbindungen aus und liefert in hohen Ausbeuten und Qualitäten in technisch einfachen Verfahrensschritten und in ökologisch günstiger Weise die entsprechenden 2-Hydroxybenzthiazol-Verbindungen.

Die nachfolgenden Beispiele dienen zur Erläuterung der Erfindung. Die Teile sind Gewichtsteile, die Prozentangaben beziehen sich auf Gewichtsprozente, sofern nichts anderes vermerkt. Gewichtsteile stehen im Verhältnis zu Volumenteilen wie Kilogramm zu Liter.

### Beispiel 1

Eine Mischung aus 150 Teilen 2-Aminobenzthiazol, 150 Teilen festem Natriumhydroxid und 300 Teilen Äthylenglykol wird 6 Stunden bei 130 bis 140° C gerührt, danach auf 80° C abgekühlt und unter Rühren in 3000 Teile Eiswasser einlaufen lassen. Nach 30 Minuten werden 5 Teile Klärkohle zugesetzt, die Lösung 15 Minuten nachgerührt und sodann filtriert. Das Filtrat wird mit 10%iger wäßriger Salzsäure auf einen pH-Wert von 5 bis 6 gestellt und der ausgefallene 2-Mercaptophenylharnstoff durch Filtration isoliert. (Das getrocknete Produkt belegt eine Ausbeute von 92% d. Th. mit einem Schmelzpunkt von 151 bis 153° C.)

Das isolierte Feuchtprodukt wird unverzüglich in 700 Teile einer 10%igen wäßrigen Salzsäure eingetragen und unter Rühren 15 Minuten lang bei 90° C erwärmt. Das hierbei ausfallende 2-Hydroxybenzthiazol wird nach Abkühlen des sauren Ansatzes durch Filtration isoliert, mit Wasser gewaschen und getrocknet. Man erhält 135 Teile eines analysenreinen Produktes entsprechend 90% d. Th., bezogen auf eingesetztes 2-Aminobenzthiazol, mit einem Schmelzpunkt von 137 bis 139° C.

### Beispiel 2

Man verfährt in der in Beispiel 1 angegebenen Verfahrensweise, setzt jedoch anstelle der 150 Teile Natriumhydroxid äquivalente Teile an festem Kaliumhydroxid oder Bariumhydroxid in die Reaktion ein. Man erhält das 2-Hydroxybenzthiazol in etwa gleich guter Ausbeute und Qualität.

### Beispiel 3

Eine Mischung aus 82 Teilen 4-Methyl-2-aminobenzthiazol, 50 Teilen festem Natriumhydroxid und 75 Teilen Glycerin wird 12 Stunden bei 130° C gerührt, sodann langsam in 1000 Teile Eiswasser einlaufen lassen. Die hierbei erhaltene Lösung des Natriumsalzes des 6-Methyl-2-mercapto-phenylharnstoffes wird nach Zusatz von 2,5 Teilen Aktivkohle geklärt. Das Filtrat wird mit 250 Teilen einer 30%igen wäßrigen Salzsäure versetzt und 15 Minuten unter Rühren bei 85 bis 90° C erhitzt, sodann auf 20° C abgekühlt. Man isoliert das ausgefallene 4-Methyl-2-hydroxy-benzthiazol durch Filtration, wäscht es mit Wasser nach und trocknet es. Man erhält 78,8 Teile eines analysenreinen Produktes entsprechend 95,5% d. Th. mit einem Schmelzpunkt von 213 bis 214° C.

### Beispiel 4

Man verfährt in der im Beispiel 3 angegebenen Verfahrensweise, ersetzt jedoch für die Ringschlußreaktion die dort verwendete Salzsäure durch eine äquivalente Menge Schwefelsäure oder Phosphorsäure. Man gelangt zu einem gleichreinen Produkt mit gleicher Ausbeute.

### Beispiel 5

Man verfährt in der im Beispiel 3 angegebenen Verfahrensweise, verwendet jedoch als Lösemittel anstelle von Glycerin eine gleiche Menge an 1,2-Dihydroxypropan oder an 1,3-Dihydroxypropan. Nach Hydrolyse wird das 4-Methyl-2-hydroxy-benzthiazol in gleich guter Ausbeute und Qualität erhalten.

### Beispiel 6

Eine Mischung aus 92,25 Teilen 6-Chlor-2-amino-benzthiazol, 60 Teilen festem Natriumhydroxyd und 150 Teilen Isobutanol wird in einem Edelstahl-Autoklaven 9 Stunden bei 140° C gerührt. Nach dem Abkühlen wird die erhaltene Suspension des Natriumsalzes des 4-Chlor-2-mercapto-phenylharnstoffes abfiltriert, der Filterrückstand mit Isobutanol gewaschen und getrocknet. Das Salz wird in einer Ausbeute von 93,2% d. Th. erhalten. Es wird in 400 Teile einer 10%igen wäßrigen Salzsäure eingetragen. Die Suspension wird 30 Minuten bei 80° C erwärmt, wobei anfangs Lösung des Salzes eintritt und nachfolgend das gebildete 6-Chlor-2-hydroxy-benzthiazol farblos ausfällt. Es wird nach Abkühlen des sauren Ansatzes filtriert, mit Wasser gewaschen und getrocknet. Man erhält 81,8 Teile, entsprechend 88,2% d. Th., eines praktisch analysenreinen Produktes mit einem Schmelzpunkt von 203 bis 204° C.

Ändert man diese Verfahrensweise in der Weise ab, daß man das isolierte Natriumsalz des 4-Chlor-2-mercapto-phenylharnstoffes isobutanolfeucht läßt und es ohne zu trocknen in die angegebene Menge Salzsäure einträgt und diese Suspension 30 Minuten bei 80° C erwärmt, so erhält man nach analoger Aufarbeitung, gegebenenfalls unter Abblasen des mit der Mercaptoverbindung eingeschleppten Isobutanols, 83 Teile 6-Chlor-2-hydroxybenzthiazol (entsprechend 89,5% d. Th.) mit einem Schmelzpunkt von 202 bis 203° C.

## Beispiel 7

Man verfährt in der im Beispiel 6 angegebenen ersten Verfahrensweise, ersetzt jedoch das Lösemittel Isobutanol durch die gleiche Menge an n-Butanol oder an Äthanol oder an Glykol-monomethyläther. Das Verfahren führt zu einem 6-Chlor-2-hydroxybenzthiazol mit etwa gleichguter Reinheit und Ausbeute.

## Beispiel 8

Eine Mischung aus 99,25 Teilen 4-Methyl-7-chlor-2-aminobenzthiazol, 100 Teilen Äthylenglykol und 60 Teilen festem Natriumhydroxyd wird 4 Stunden unter Rühren bei 160 bis 165° C erhitzt. Anschließend kühlt man unter Rühren auf eine Temperatur von 20 bis 30° C ab und isoliert das ausgefallene Natriumsalz des 6-Methyl-3-chlor-2-mercapto-phenylharnstoffes durch Absaugen. Das glykolische Filtrat dient als Vorlage für den nächsten Reaktionsansatz.

Der Filterrückstand wird mit 20 Teilen Glykol gewaschen und sodann in 300 Teile einer 10%igen wäßrigen Salzsäure eingetragen. Diese Mischung wird 60 Minuten unter Rühren bei 80 bis 85° C erhitzt. Nach dem Abkühlen isoliert man das ausgefallene 7-Chlor-4-methyl-2-hydroxy-benzthiazol durch Filtration, wäscht es mit Wasser neutral und trocknet es. Man erhält 62,4 Teile (entsprechend 62,5% d. Th.) eines analysenreinen Produktes mit einem Schmelzpunkt von 265,5 bis 266° C.

Wird die glykolische Reaktionsmischung entsprechend Beispiel 1 aufgearbeitet, so erhält man das 7-Chlor-4-methyl-2-hydroxy-benzthiazol in einer Ausbeute von 85,6% d. Th. und praktisch gleicher Reinheit.

## Beispiel 9

Eine Mischung aus 99,25 Teilen 4-Methyl-7-chlor-2-amino-benzthiazol und 20 Teilen festem Natriumhydroxid und der genannten glykolischen Mutterlauge aus Beispiel 8 wird 4 Stunden unter Rühren bei 160 bis 165° C erhitzt. Den Ansatz kühlt man sodann unter Rühren auf eine Temperatur von 20 bis 30° C ab und verfährt sodann zur Cyclisierung des ausgefallenen Natriumsalzes des 6-Methyl-3-chlor-2-mercapto-phenylharnstoffes gemäß den Angaben des Beispieles 8 weiter. Man erhält 85 Teile 7-Chlor-4-methyl-2-hydroxy-benzthiazol mit einem Schmelzpunkt von 265,5 bis 266° C entsprechend einer Ausbeute von 85,2% d. Th.

## Beispiel 10

Man verfährt wie im Beispiel 8 und nachfolgend im Beispiel 9 angegeben, ersetzt jedoch die Ausgangsverbindung 4-Methyl-7-chlor-2-amino-benzthiazol durch die äquivalente Menge an 4-Chlor-2-amino-benzthiazol. Man erhält über die Zwischenstufe des Natriumsalzes des 6-Chlor-2-mercapto-phenylharnstoffes als Endprodukt das 4-Chlor-2-hydroxy-benzthiazol in einer Ausbeute von 59,3% d. Th. gemäß der Arbeitsweise des Beispieles 8 und in einer Ausbeute von 84,0% d. Th. gemäß der Verfahrensweise des Beispieles 9, jeweils mit einem Schmelzpunkt von 204,5 bis 205° C.

## Beispiele 11 bis 19

Verfährt man in erfindungsgemäßer Weise zur Herstellung von 2-Hydroxy-benzthiazol-Verbindungen, beispielsweise in analoger Weise, wie in den obigen Ausführungsbeispielen beschrieben, und geht hierbei von 2-Amino-benzthiazol-Verbindungen entsprechend der allgemeinen Formel (1a) mit den in den nachfolgenden Tabellenbeispielen angegebenen Resten $R_1$ und $R_2$ aus, so erhält man die entsprechenden 2-Hydroxy-benzthiazol-Verbindungen entsprechend der allgemeinen Formel (4a) mit

den in den Tabellenbeispielen angegebenen Substituenten $R_1$ und $R_2$ mit den dort ebenfalls angegebenen Ausbeuten und Schmelzpunkten.

| Beispiel | Verbindung (1a) $R_1$ | $R_2$ | Verbindung (4a) $R_1$ | $R_2$ | Ausbeute (% d. Th.) | Smp. (°C) |
|---|---|---|---|---|---|---|
| 11 | 6-Methyl | H | 6-Methyl | H | 89,1 | 168,5—170 |
| 12 | 6-Brom | H | 6-Brom | H | 79,4 | 222—223 |
| 13 | 4-Methyl | 6-Chlor | 4-Methyl | 6-Chlor | 85,7 | 243,5—224 |
| 14 | 6-Hydroxy | H | 6-Hydroxy | H | 79,2 | 240—242 |
| 15 | 6-Methoxy | H | 6-Methoxy | H | 84,3 | 162,5—164 |
| 16 | 4-Methoxy | H | 4-Methoxy | H | 78,7 | 170—171,5 |
| 17 | 6-Äthoxy | H | 6-Äthoxy | H | 86,9 | 145,5—146,5 |
| 18 | 4-Methyl | 6-Methyl | 4-Methyl | 6-Methyl | 88,4 | 216—217 |
| 19 | 6-Sulfo | H | 6-Sulfo | H | 77,4 | > 300 |

**Patentansprüche**

1. Verfahren zur Herstellung von 2-Hydroxy-benzthiazolen, dadurch gekennzeichnet, daß man eine 2-Amino-benzthiazol-Verbindung mit einem Alkali- oder Erdalkalihydroxid in zumindest stöchiometrischer Menge in einem alkalibeständigen Löse- oder Verdünnungsmittel in Abwesenheit von Wasser oder lediglich in Anwesenheit von höchstens 2,5 Gew.-% Wasser in der Reaktionsmischung, bezogen auf die Reaktionsmischung, behandelt, das sich bildende Alkali- oder Erdalkalisalz der ortho-Mercapto-N-phenylharnstoff-Verbindung, gegebenenfalls ohne deren Zwischenisolierung, durch Säure in deren ortho-Thiophenolharnstoff-Verbindung überführt und diese mittels Säure bei einer Temperatur zwischen 40 und 100° C zur 2-Hydroxy-benzthiazol-Verbindung cyclisiert.

2. Verfahren nach Anspruch 1, dadurch gekennzeichnet, daß man als alkalibeständige Löse- und Verdünnungsmittel ein- oder mehrwertige aliphatische Alkohole und deren niedere Monoalkyläther verwendet.

3. Verfahren nach Anspruch 1 oder 2, dadurch gekennzeichnet, daß die Umsetzung der 2-Amino-benzthiazol-Verbindung mit einem Alkali- oder Erdalkalihydroxid zum entsprechenden Salz der ortho-Mercapto-N-phenyl-harnstoff-Verbindung bei einer Temperatur zwischen 120 und 160° C erfolgt.

4. Verfahren nach einem der Ansprüche 1 bis 3, dadurch gekennzeichnet, daß die Cyclisierungsreaktion bei einer Temperatur zwischen 60 und 90° C erfolgt.

5. Verfahren nach einem der Ansprüche 1 bis 4, dadurch gekennzeichnet, daß das alkalibeständige Löse- und Verdünnungsmittel ein Alkanol von 1 bis 6 C-Atomen ist.

6. Verfahren nach einem der Ansprüche 1 bis 4, dadurch gekennzeichnet, daß das alkalibeständige Löse- und Verdünnungsmittel ein Alkanglykol von 2 bis 5 C-Atomen oder ein Alkantriol von 3 bis 8 C-Atomen oder ein niederer Monoalkyläther solcher Glykole und Triole ist.

7. Verfahren nach einem der Ansprüche 1 bis 6, nach welchem eine 2-Amino-benzthiazol-Verbindung der allgemeinen Formel (1a)

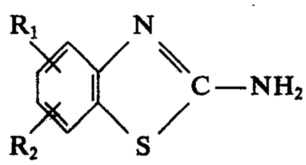

(1a)

in welcher $R_1$ und $R_2$ gleich oder verschieden voneinander sind und jedes ein Wasserstoffatom, eine Alkylgruppe, eine Alkoxygruppe, ein Halogenatom, eine Hydroxygruppe oder eine Sulfogruppe bedeuten, zu einer 2-Hydroxy-benzthiazol-Verbindung der allgemeinen Formel (4a)

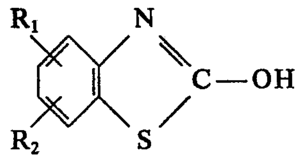

(4a)

in welcher $R_1$ und $R_2$ die obengenannten Bedeutungen haben, umsetzt.

**Claims**

1. Process for the manufacture of 2-hydroxy-benzothiazole compounds, characterized in that a 2-amino-benzothiazole compound is treated with an alkali metal hydroxide or alkaline earth metal hydroxide in at least the stoichiometric amount in a solvent or diluent stable to alkalis, in the absence of water or in the presence only of 2.5% by weight at most of water in the reaction mixture, calculated on the reaction mixture, and that the alkali metal or alkaline earth metal salt of the ortho-mercapto-N-phenylurea compound being formed is converted, optionally without its intermediate isolation, by an acid into its ortho-thiophenyl-urea compound which is then cyclized to the 2-hydroxy-benzothiazole compound by means of an acid at a temperature between 40 and 100° C.

2. Process according to claim 1, characterized in that mono- or polyvalent aliphatic alcohols or their lower monoalkyl ethers are used as solvents and diluents stable to alkalis.

3. Process according to claim 1 or 2, characterized in that the reaction of the 2-amino-benzothiazole compound with an alkali metal hydroxide or alkaline earth metal hydroxide, to give the corresponding salt of the ortho-mercapto-N-phenylurea compound, is conducted at a temperature between 120 and 160° C.

4. Process according to one of claims 1 to 3, characterized in that the cyclization reaction is conducted at a temperature between 60 and 90° C.

5. Process according to one of claims 1 to 4, characterized in that the solvent and diluent stable to alkalis is an alkanol of from 1 to 6 C-atoms.

6. Process according to one of claims 1 to 4, characterized in that the solvent and diluent stable to alkalis is an alkane glycole of from 2 to 5 C-atoms or an alkane triol of from 3 to 8 C-atoms or a lower monoalkylether of those glycoles and triols.

7. Process according to one of claims 1 to 6, in which a 2-amino-benzothiazole compound of the general formula (1a)

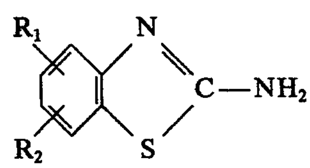

(1a)

in which $R_1$ and $R_2$ are identical to or different from one another and each is a hydrogen atom, an alkyl group, an alkoxy group, a halogen atom, a hydroxy group or a sulfo group, is reacted to give a 2-hydroxy-benzothiazole compound of the general formula (4a)

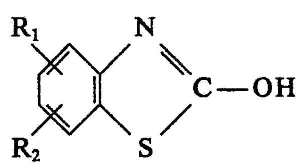

(4a)

in which $R_1$ and $R_2$ have the above mentioned meanings.

**Revendications**

1. Procédé pour préparer des hydroxy-2 benzothiazoles, caractérisé en ce qu'on traite un amino-2 benzothiazole par une quantité au moins stoechiométrique d'un hydroxyde alcalin ou alcalino-terreux dans un solvant ou diluant stable en milieu alcalin, en l'absence d'eau ou tout simplement en présence d'au maximum 2,5% en poids d'eau dans le mélange réactionnel, par rapport à ce mélange réactionnel, on transforme, éventuellement sans son isolement intermédiaire, le sel alcalin ou alcalino-terreux de l'ortho-mercapto-N-phénylurée qui se forme par de l'acide en l'ortho-thiophénolurée correspondante que l'on cyclise au moyen d'un acide, à une température comprise entre 40 et 100°C, pour obtenir le composé de type hydroxy-2 benzothiazole.

2. Procédé selon la revendication 1, caractérisé en ce qu'on utilise comme solvant et diluant pouvant résister aux substances alcalines des mono- ou poly-alcools aliphatiques et leurs esters monoalkyliques inférieurs.

3. Procédé selon la revendication 1 ou 2, caractérisé en ce que la réaction de l'amino-2 benzothiazole avec un hydroxyde alcalin ou alcalino-terreux pour donner le sel correspondant de l'ortho-mercapto-N-phénylurée s'effectue à une température comprise entre 120 et 160° C.

4. Procédé selon l'une des revendications 1 à 3, caractérisé en ce que la réaction de cyclisation est effectuée à une température comprise entre 60 et 90° C.

5. Procédé selon l'une des revendications 1 à 4, caractérisé en ce que le solvant ou diluant, stable en milieu alcalin, est un alcanol ayant 1 à 6 atomes de carbone.

6. Procédé selon l'une des revendications 1 à 4, caractérisé en ce que le solvant ou diluant stable en milieu alcalin est un alcane-glycol ayant 2 à 5 atomes de carbone ou un alcane-triol ayant 3 à 8 atomes de carbone ou un éther monoalkylique inférieur de tels glycols et triols.

7. Procédé selon l'une des revendications 1 à 6, selon lequel on fait réagir un amino-2 benzothiazole de formule générale (1a):

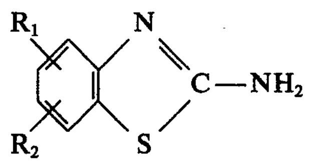

(1a)

[dans laquelle $R_1$ et $R_2$ sont identiques ou différents et représentent chacun un atome d'hydrogène, un groupe alkyle, un groupe alcoxy, un atome d'halogène, un groupe hydroxyle ou un groupe sulfo] pour obtenir un hydroxy-2 benzothiazole de formule générale (4a):

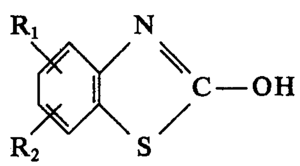

(4a)

dans laquelle $R_1$ et $R_2$ ont les sens indiqués ci-dessus.